# EUROPEAN PATENT SPECIFICATION

(11) **EP 0 090 577 B1**
(45) Date of publication and mention of the grant of the patent: **28.05.1986**
(21) Application number: 83301581.1
(22) Date of filing: 22.03.1983
(51) Int. Cl.: A01N 55/00, C08G 77/26

(54) **Insoluble polymeric contact preservatives**
Unlösliche polymere Konservierungsmittel
Polymères insolubles comme agents de conservation

(30) Priority: 24.03.1982 US 361277
(43) Date of publication of application: 05.10.1983
(73) Proprietor: DOW CORNING CORPORATION, Midland Michigan 48640 (US)
(72) Inventor: Isquith, Alan Jay, Midland Michigan (US); Gettings, Richard Lee, Freeland Michigan (US)
(74) Representative: Laredo, Jack Joseph

## Description

This invention deals with a novel silsesquioxane and its use to inhibit the growth of microorganisms.

It is well known that certain quaternary salts have solution antimicrobial activity and that such salts are active against a fairly broad spectrum of bacteria, fungi, algae and yeasts. The quaternary salts are only a part of a very large collection of soluble reactive chemicals used in many varied applications.

An alternative method used for sterilization preservation or other modes of microbial inhibition has just recently been discovered and has been termed a "surface-bonded" antimicrobial method. Since 1969, there has been a great deal of work in this area of surface bonded functional materials and the published art is fairly extensive. An example of published art in this area is, for instance, "Surface-bonded Antimicrobial Activity of an Organosilicon Quaternary Ammonium Chloride", Isquith, A. J. Abbott, E. A. and Walters, P. A., Appl. Microbiol. 24(6):859-863 (1972).

In addition, there exists the physical processes for inhibiting microbial growth such as, for example, heat, ultraviolet and ionizing radiation and the filtration of the microorganisms by membrane filtration of quantitative sorption on a surface active material packed in a bed.

A relatively new approach to the control of microorganisms has been to use insoluble contact antimicrobial materials such as is described in "Prolongation of the Antibacterial Potential of Disinfected Surfaces", Klarmann, E. G., Wright, E. S., and Shternov, V. A., Appl. Microbiol. 1:19-23 (1953) and Janauer, G. E. et al, "Insoluble Polymeric Contact Disinfectants: An Alternative Approach to Water Disinfection", Chemistry in Water Rinse, 1:501-521, (1981).

Janauer describes this method in terms of the antimicrobial material as being a material that can inactivate or kill target microorganisms by mere contact without adding any reactive agent to the bulk phase being disinfected and further, that the material does not become irreversibly inactivated for further use. The approach, as described by Janauer, is based on inert polymeric materials which allow the incorporation or permanent attachment of germicidal moieties.

Janauer, in particular points out the advantages of this type of approach to the varied problems and suggests the following criteria for any truly useful system. The material should be completely water insoluble and the material should not be reactive with its surroundings such that it is rendered inactive. The material should give instant kill. It should be odorless and tasteless. It should be a broad-spectrum germicide. The biofunctional sites must be easily accessible. The material should retain the germicidal activity for a long time and should not be subject to "exhaustion". It should resist fouling by diverse inorganic and organic matter.

Prior to Janauer's disclosure, the next best thing to truly insoluble/nonreactive disinfectants were the "demand-release" disinfectants represented by macroreticular resin compositions impregnated with a silver salt of very low solubility. In spite of the low solubility of the silver salt and means to further reduce the leaching and solubilizing of the silver salt, these compounds add toxic silver to the influents being treated.

Janauer, therefore, discloses what he believes to be a truly insoluble polymeric contact disinfectant concept which is the treatment of halomethylated microporous polymers with tertiary amines containing a higher alkyl side chain similar to those found in solution active quaternary salts.

Such a material was described by Janauer as the reaction product of halomethylated polystyrene and N,N-dimethyldodecylamine.

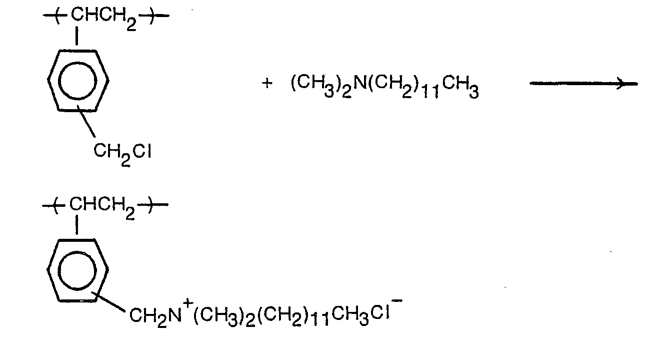

One of the major problems cited by Janauer is the inconsistency of the materials produced by this method probably owing to the fact that only 50-60% of the chloromethyl groups are aminated and the interior surfaces of the reticulated polymer have "too much functionalization of the resin interior ... (and) a highly functionalized resin may show slower flow rate characteristics".

Because of the problems associated with prior art systems, the inventors herein have developed new and novel insoluble contact antimicrobials having use beyond the treatment of water as described by Janauer.

The instant invention consists of novel insoluble contact antimicrobial materials comprising silsesquioxanes and their use as antimicrobial agents.

What is disclosed, therefore, are silsesquioxanes having the unit formula 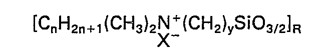 wherein n has a value of 8 to 22, y has a value of 1 to 4, R has a value such that the silsesquioxane is an insoluble, friable solid and, X is chloride, bromide or carboxylate radical. What is further disclosed'is the use of the silsesquioxanes in a method of inhibiting the growth of microorganisms which method consists of inhibiting the growth of microorganisms by contacting said microorganisms with a polymeric silsesquioxane having the unit formula 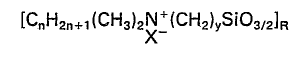 where n has a value of 8 to 22, y has a value of 1 to 4, R has a value such that the silsesquioxane is an insoluble, friable solid and, X is chloride, bromide or carboxylate radical.

These silsesquioxanes may be used as such as insoluble contact antimicrobial materials.

They may also be used in a cream base, either with or without a medicament. The instant invention, therefore, consists of silsesquioxanes wherein the alkyl substitution on the nitrogen atoms other than two methyl groups consists of CₙH₂ₙ₊₁ wherein n has a value of 8-22. Preferred for this invention are silsesquioxanes having such an alkyl group wherein n has a value of at least 12. Most preferred for this invention is the silsesquioxane wherein the alkyl group is C₁₈H₃₇. As shown above, y has a value of 1-4. Preferred are those compounds wherein y has a value of 3 or 4. The most preferred compounds are those wherein y has a value of 3.

As noted above, the anion of such a silsesquioxane can be chloride, bromide or carboxylate radical. Preferred for this invention are the halides, chlorine and bromine. Most preferred is the chloride ion.

Also as noted above, the value of R is such that the silsesquioxane is insoluble and a friable solid. By insoluble, it is meant that the silsesquioxanes are insoluble in water as well as solvents. By being insoluble in solvents, the silsesquioxanes can be made in non-aqueous systems. Obviously, since the silsesquioxanes are not soluble in solvents, they are incapable of being analyzed to ascertain their exact structure: however, knowledge of the exact structure of the silsesquioxane is not critical to this invention as long as the silsesquioxane is insoluble and friable.

FR-A-2218052 discloses the use of polymeric organosiloxane compounds having the unit formula 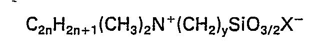 to coat inert solids in particular polymeric matrices containing solid fillers, in order to inhibit the growth of microorganisms brought into contact with the resulting coated substrates. As indicated below such antimicrobial composites have the disadvantage inherent in the use of the antimicrobial material and specifically the quaternary moiety thereof as a coating on a substrate. In practice this has been found to have the disadvantage of immobilising the antimicrobial moiety on the inert support with the result that the antimicrobial. action is only exerted in close proximity to the inert support. In contrast, the disadvantages of this prior art teaching are overcome in the simplest possible way by the present invention according to which the silsesquioxanes which incorporate therein the quarternary moiety having the antimicrobial activity are themselves used as their own substrate, namely, as the insoluble contact antimicrobial materials without the intervention of any additional material or the need for use of the silsesquioxanes as coatings or otherwise to form composites as in the prior art.

The silsesquioxanes are prepared by known methods. Several methods, for example, are shown in Eaborn, C., "Organosilicon Compounds", Butterworths Scientific Publications, pp. 263-264, (1960). In general, the precursor silane, for example, 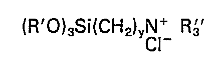 in which R3' corresponds to CₙH₂ₙ₊₁(CH₃)₂ as defined above is prepared from (R'O)₃Si(CH₂)_{y}Cl and NR3' and then the silane is hydrolyzed in solvent to by-produce an alcohol, corresponding to the (R'O)₃-moiety, and the silanol (HO)₃SI(CH₂)yN⁺ R3' CI- which silanol condenses rapidly to increase in molecular weight and form the silsesquioxane. The silsesquioxane is continually heated to further increase the material in molecular weight until a friable solid is obtained. It is such a solid which is the subject of this invention. Because of the insolubility of such a solid, the solid "pops" out of the solution as a slippery mass which upon further work-up becomes, white, crystalline-appearing friable particles. In this form the materials are ready for use in inhibiting microorganisms.

The use of these materials for inhibiting microorganisms has great value in the preservation of cosmetics and cream based medicaments. The use of quaternary compounds to concentrate antimicrobial activity at the desired site of action, thus minimizing the amount of chemical agent needed and thus preventing adsorption in humans and/or dispersion in the environment has been advantageous. The disadvantage of this kind of immobilization on inert supports has been the use of such compounds only against microorganisms in close proximity to the inert support. This disadvantage can be overcome by utilizing the silsesquioxanes of the instant invention since the silsesquioxanes of the instant invention are their own substrate. These materials are non-absorbable on either oral or dermal exposure in animals. They have wide spectrum antimicrobial activity, particularly against Pseudomonads, they are nontoxic, nonirritating and nonsensitizing. They are odorless, colorless and stable over a wide range of storage conditions. They are easily incorporated in the formulations for cosmetics, burn ointments, ophthalmic solutions and cream based medicaments.

### Example 1

### Preparation of a silsesquioxane.

A sample of crude 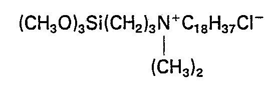 was stripped under vacuum and recrystallized from hot hexane. This sample (106 gms) was then placed on a rotary evaporator and heated to 90°C for three hours. A creamy solid resulted. This solid was dissolved in hot hexane and recrystallized a total of 4 times and three separate crops of the quaternary salt were obtained. This material was a white solid.

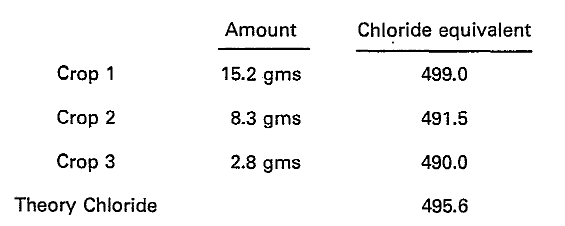

The above material was dissolved in an equal weight of acetone. Distilled water (300% theory) was added and the mixture refluxed for three hours. A waxy solid formed upon cooling which was filtered from the mixture, rinsed with acetone and air dried. A total of 14.28 gms of cream-colored powder was recovered, a yield of 76% based on a molecular weight of 427 for the hydrolysate. This material was insoluble at a level of 1% in ether, acetone, isopropanol, ethanol, water, toluene, and methyl isobutyl ketone at room temperature. Some limited solubility was apparent in water. A portion (4.4521 gms) of this material was then placed in a Soxhlet extractor for eight hours with 75 mls of distilled water. A total of 3.4091 gms (77%) of water insoluble powder remained in the thimble, and 0.6518 gms of solid was recovered from the water after vacuum evaporation. A second Soxhlet extraction of this material with distilled water resulted in a 96% recovery (3.2621 gms) of the water insoluble powder.

Formulations unless specifically produced as sterile products generally contain microorganisms which under most storage conditions can grow and multiply resulting in either an unsafe product (health hazard) or spoilage of the product. Organisms of the genus Pseudomonas, found in a large number of products, are the causative agent of many of these deleterious effects. Compounds described in this invention are particularly active in inhibiting the growth of Pseudomonads.

### Example 2

### Determination of Adequacy of Preservation in a Standard Nonionic Cream.

Varying amounts of [C₁₈H₃₇(CH₃)₂N+(CH₂)₃SIO_{3/2}Cl⁻]_{R} as prepared above were added to a standard nonionic hand cream for the purpose of testing the preservation adequacy. The following were weighed into a glass beaker:

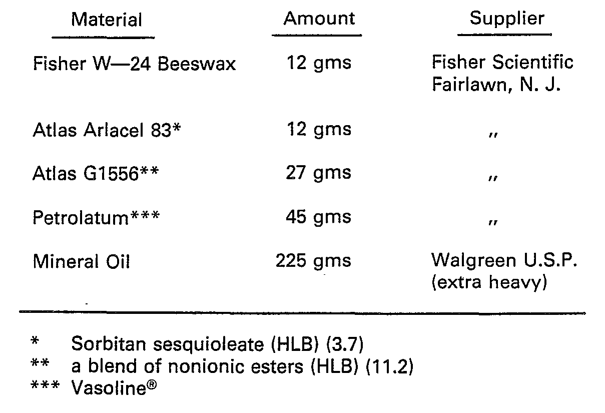

These materials were heated and stirred at 65°C using a magnetic stirrer plate. The antimicrobial was stirred into this part. This combination is part I. A second part (II) was 300 gms of distilled hot water (65°C).

Twenty three gms of part II at 65°C were added to 26.7 gms of 65°C part I with rapid stirring. This mixture was stirred until the temperature reached 40°C at which point it was allowed to cool to room temperature without stirring. All test formulations were prepared in this manner.

Twenty milliliter aliquots of each test formulation were challenged with approximately one million cells of Pseudomonas aeruginosa PRD-10 (ATCC#15442) per ml. of formulation. After adding the inoculum, the samples were mixed thoroughly. A one ml. aliquot from each sample was removed, diluted in suitable neutralizing broth, and plated to establish a base line bacterial count at time zero. The samples were stored at 37°C and sampled at 3, 7, 14, 21 and 28 days to determine the number of viable organisms remaining in each sample. Table I shows the amount of additive used. Table II shows the results.

Thus, it can be observed that under the conditions employed, concentrations of the compound described ranging from 0.01 to 1.00 weight percent acted as an effective preservative in preventing the growth of Pseudomonas.

### Example 3

Microbial Recovery from Nonionic Hand Creams Preserved with 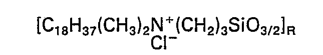 and Challenged with Various Strains of Pseudomonas aeruginosa.

Various strains of Pseudomonas aeruginosa in a nonionic hand cream as prepared above were tested. The antimicrobial agent was the material prepared in example 1 and was used at 0.33 weight percent based on total solids in the formulation. The results are shown on table III. It can be observed that the antimicrobial agent is effective as a preservative in the nonionic cream against several strains of Pseudomonas aeruginosa.

### Example 4

### Microbial Recovery from Nonionic Hand Creams Containing Various Preservation Agents and Challenged with Pseudomonas aeruginosa ATCC 19660.

Table IV shows the results of using several antimicrobial agents of this invention as preservatives in nonionic hand creams. The antimicrobial agents containing dodecyl, tetradecyl and hexadecyl radicals were tested against Pseudomonas aeruginosa ATCC 19660 with good preservative results. The testing was carried out similar to that in examples 2 and 3.

### Example 5

Preservative action is generally dependent on the solubility of the preservative in the aqueous phase (Rosen, W.E., et. al., "Modern Concepts of Cosmetic Preservation". J. Soc. Cosmet. Chem., 24, 663-675 (1973)). The inventive compositions herein are not soluble in water and/or oils. They represent a combination lipophilic/hydrophilic polymer which, although not soluble in either phase, can function as a dispersed particle in either phase. Because of this attribute, the materials are useful in such applications as preservatives in cosmetics, food and feed. These materials are also useful in therapeutic applications such as burn medication, skin antiseptics, post-operative eye medication, antidandruff and athletes foot preparations, soaps, preoperative gastrointestinal sterilization, deodorants, acne preparations, surgical scrubs, laundry detergents, toothpastes, preservation of oils and veterinary care products.

Along these lines of applications, the agents of this invention were tested in the following examples. The antimicrobial agents used herein were prepared as in examples 2 and 3. Materials currently used as preservatives that are commercially available were tested in comparison studies. Methyl and propyl parabens (para-hydroxybenzoates) were obtained from D-R Chemical Company, Detroit, Michigan, U.S.A. Silvadene® (containing 1% silver sulfadiazine) was obtained from Marion Laboratories Kansas City, Mo. PhisohexdTM (3% hexachlorophene) was purchased from Winthrop Laboratories; New York, New York.

The antimicrobial agents of this invention as prepared in example 1 and the parabens were tested in a preservative efficacy test by incorporating them into the oil-in-water, non-ionic hand cream formulation of example 2. In this example, forty grams of each test and control formulation containing no additives for preservation were challenged with standardized (1 x 10⁶ organisms) pure cultures of Pseudomonas aeruginosa ATCC 15442, Pseudomonas aeruginosa ATCC 19660, Staphylococcus aureus ATCC 6538, Candida albicans ATCC 10231 and Aspergillus niger ATCC 6275. The formulations were subsampled periodically for 28 days to determine the number of surviving microorganisms.

The activity of conventional organic quaternary ammonium compounds is generally accepted as reduced against Pseudomonads. Although attractive because of their low toxicity, this weakness in their spectrum of activity has limited the use of organic quaternaries as preservatives in formulations attacked by these organisms. The agents of this invention by contrast, were highly effective against Pseudomonas aeruginosa ATCC 15442 in oil-in-water, non-ionic formulations while retaining antimicrobial activity against staphylococci, yeast, and fungi. Activity of the inventive agents against Staphylococcus aureus ATCC 6538 was identified at concentrations of 0.33 and 1.0 weight percent. See Tables V to VIII.

Preservative challenge tests conducted with the inventive agents of varying alkyl chain length indicate that the C₁₈H₃₇ substituted agent may be the least active of the series tested. These tests in an oil-in-water ointment indicate a more rapid rate of kill against Pseudomonas aeruginosa ATCC 19660 was achieved with the C₁₂H₂₅ and C₁₄H₂₉ agents.
(A) [Cl⁻(CH₃)₂N⁺(CH₂)₃SiO_{3/2}]_{R};
(B) [Cl⁻(CH₃)₂(C₁₂H₂₅)N⁺(CH₂)₃SiO_{3/2}]_{R};
(C) [Cl⁻(CH₃)₂(C₁₄H₂₉)N⁺(CH₂)₃SiO_{3/2}]_{R};
(D) [Cl⁻(CH₃)₂(C₁₆H₃₃)N⁺(CH₂)₃SIO_{3/2}]_{R}; and
(E) [Cl⁻(CH₃)₂(C₁₈H₃₇)N⁺(CH₂)₃SiO_{3/2}]_{R}. See Table IX.

### Example 6

### Burn Therapy

The method used to study local therapy of Pseudomonas septicemia in burned mice was similar to that used by Rosenthal "Local and Systemic therapy of Pseudomonas Septicemia in Burned Mice, Annals of Surgery 165: 97-103 (1967). In this preliminary investigation, the antimicrobial agents of this invention were incorporated into a hydrophilic oil-in-water ointment base for application (22% stearyl alcohol, 25% petrolatum, 2% sorbitan monoleate, 1% isopropyl myristate, 12% propylene glycol, 5% polyoxyl 40 stearate, and 33% water).

White mice, strain CFW-1 (Carworth, Inc.), Portage, Michigan, were anesthetized with PenthraneTM (methoxyflurane) manufactured by Abbott laboratories, Chicago, Illinois in a closed chamber. The tail and lower part of the thigh region (approximately 25% of the body surface) were immersed in 70°C water for five seconds. The tail was subsequently challenged with a standardized culture of Pseudomonas aeruginosa ATCC 19660 two hours postburn. Local therapy to the burned area was started six hours postburn and continued for two consecutive days, once per day. The first treatment only was applied to anesthetized mice to allow for longer contact. Mice were observed each day for seven days, deaths recorded, and organ cultures completed from dead animals to confirm Pseudomonas septicemia.

Preliminary evaluation of the antimicrobial agent of this invention into a non-ionic ointment indicates activity equivalent to silver sufadiazine in an in vivo test system against a Pseudomonad. Deaths recorded were confirmed by isolation of the test organism from internal organs of necropsied animals. See Table X.

### Example 7

### Surgical Scrub Efficiency Test

Potential activity in skin antisepsis was evaluated by the Glove Juice Test as described by Engley and Dey, Engley, F. et. al. Proceedings of the Chem. Spec. Mfg. Assoc. (CSMA), Mid-year meeting (1970) on twenty adult subjects. Following a two week pre-test period where subjects avoided hand contact with antimicrobial or detergent formulations, base-line microbiological counts were established over a one week sampling period. For the actual test, subjects were randomly divided into two groups. One group scrubbed with Phisohex (3 wt. percent hexachlorophene) and the other with 3 wt. percent 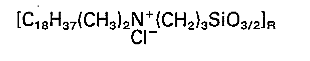 in Methocel. Each subject served as their own control by washing the opposite hand under the same conditions with Camay bar soap. Bacterial sampling of the gloved hands, utilizing a neutralizer stripping solution, was at various prescribed time intervals. The test was performed following a single scrub procedure.

The data expressed in Table XI below indicates potential utility of the 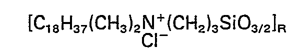 as a disinfectant or antiseptic scrub. The actual reduction in microbial counts achieved by both the inventive agent and hexachlorophene are not impressive, however, the data were collected following a single exposure (scrub) to the agents.

"The American Type Culture Collection, Catalogue of Strains 1, fifteenth Edition 1982 lists the following strains: Pseudomonas aeruginosa PRD-10 ATCC 15442, page 168; Pseudomonas aeruginosa ATCC 19660, page 168; Staphylococcus aureus ATCC 6538, page 194; Candida albicans ATCC 10231, page 305; Aspergillus niger ATCC 6275, page 289".

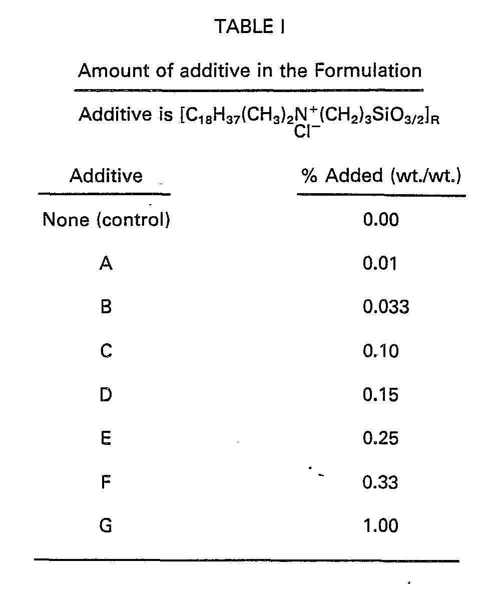

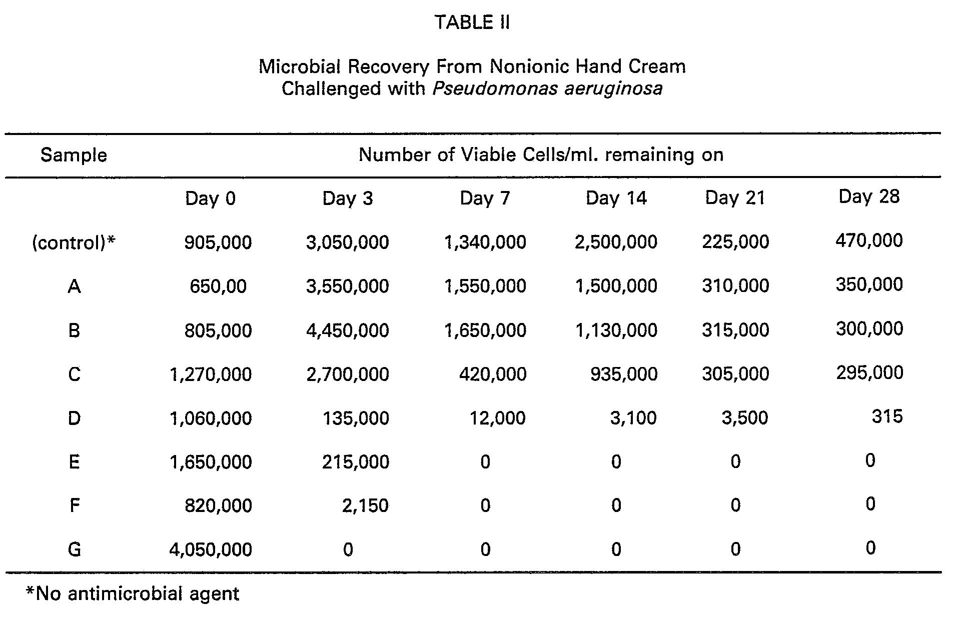

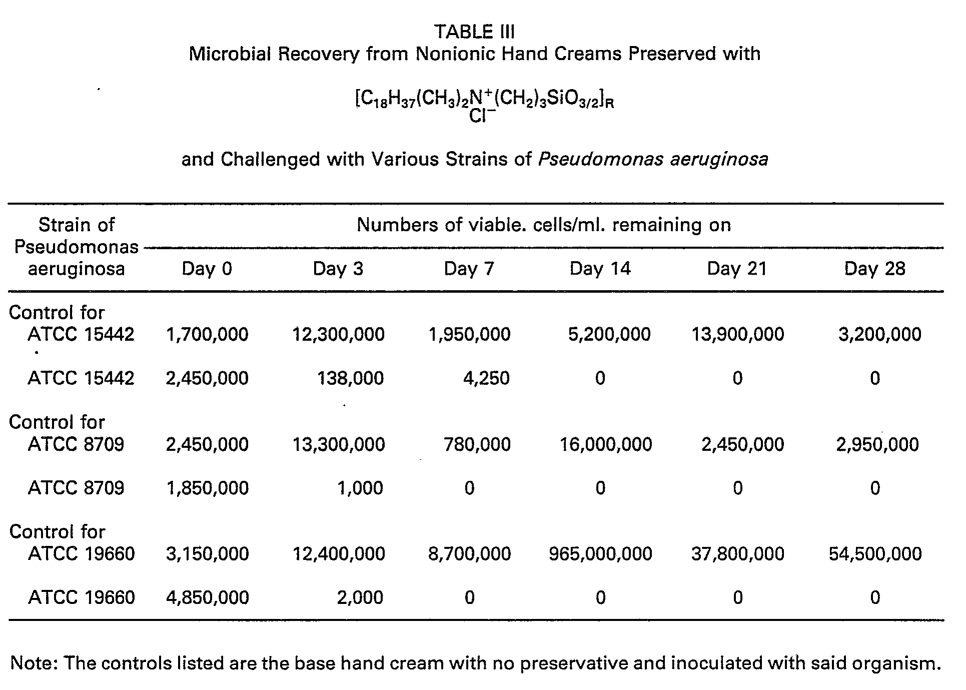

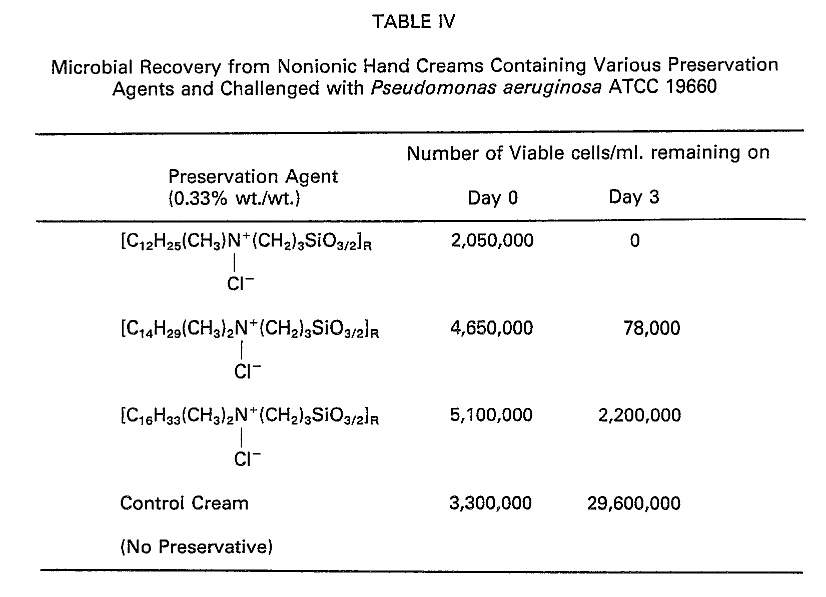

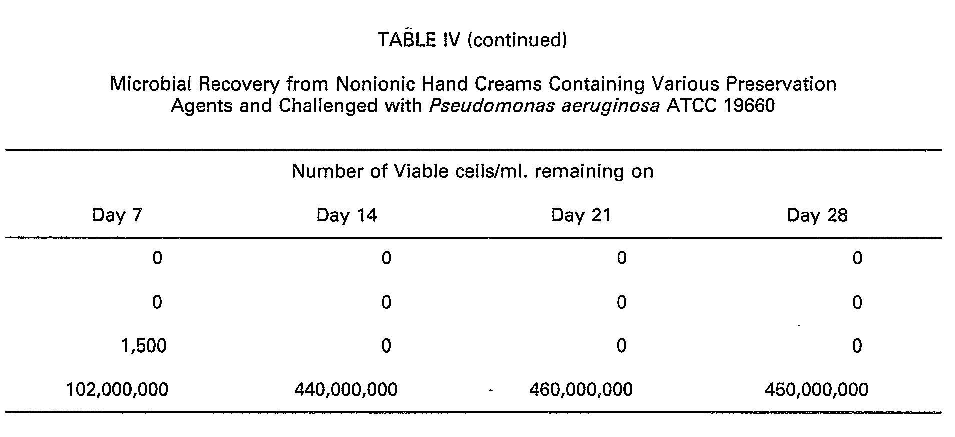

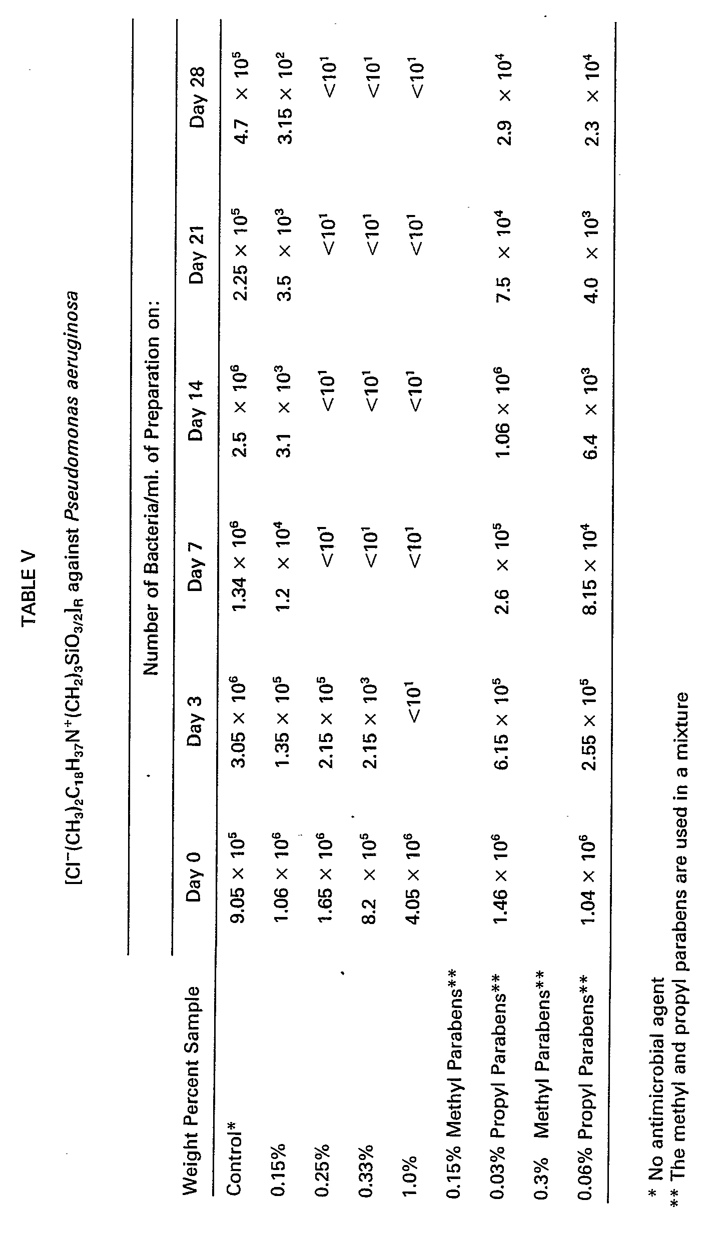

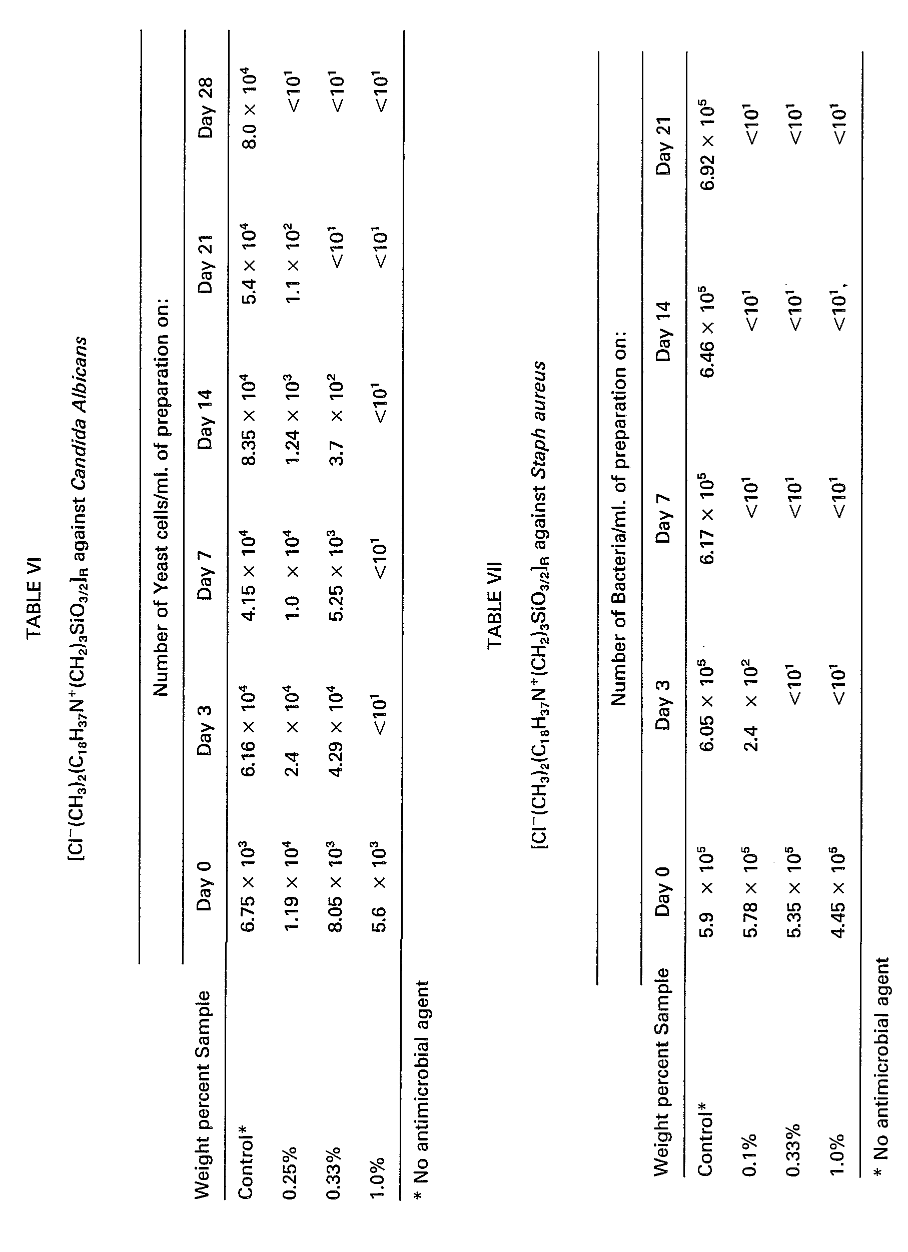

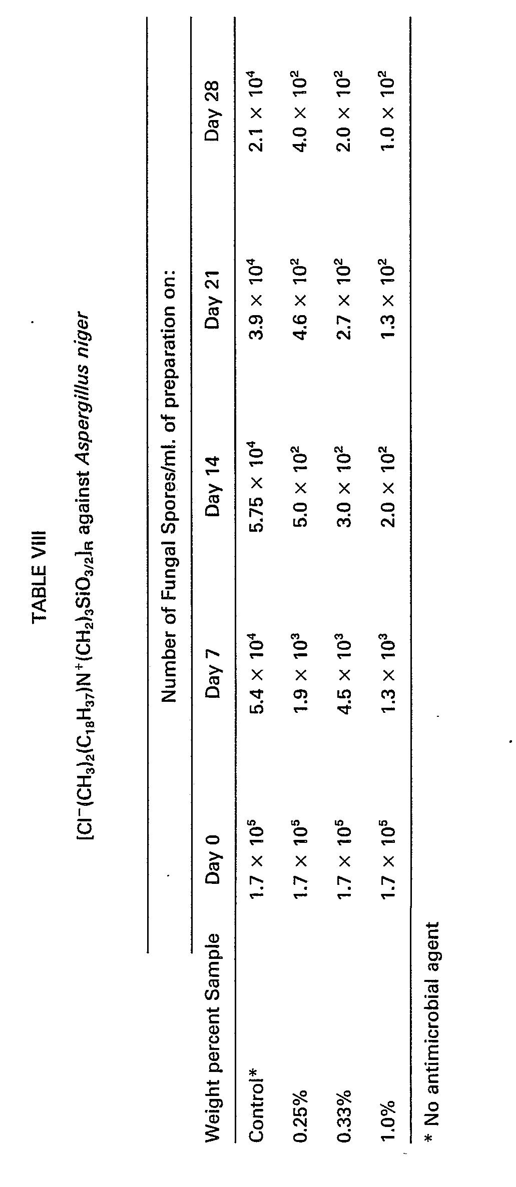

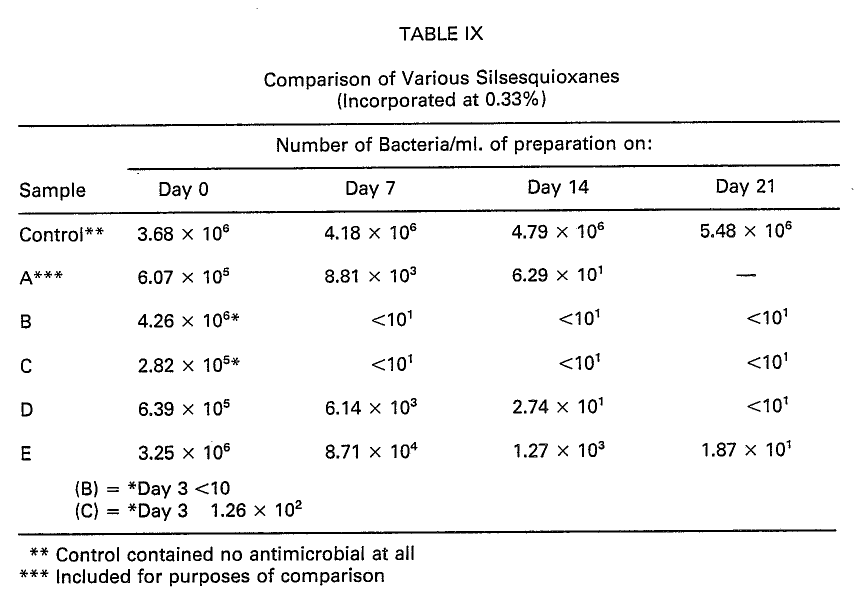

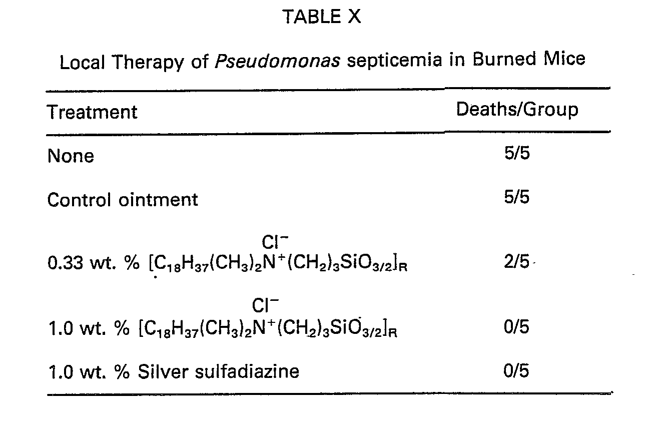

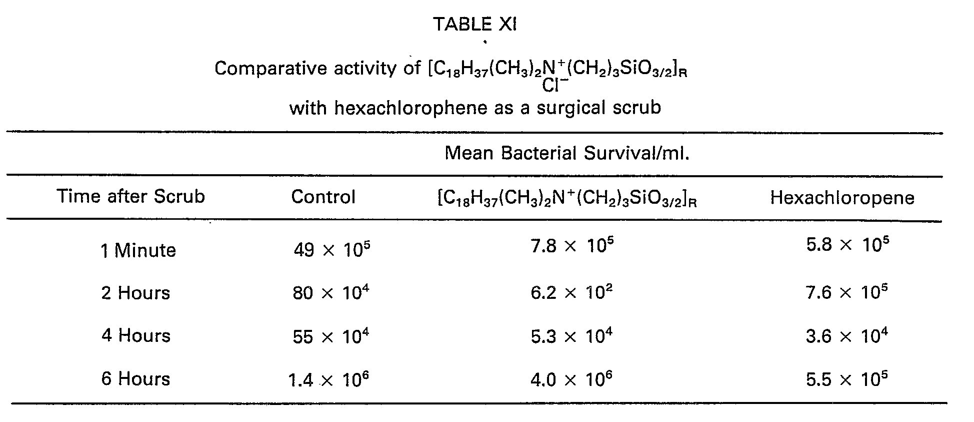

## Claims

1. An insoluble contact antimicrobial material comprising a silsesquioxane having the unit formula 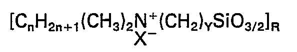 wherein n has a value of 8 to 22, y has a value of 1 to 4, R has a value such that the silsesquioxane is an insoluble, friable solid and X is chloride, bromide or carboxylate radical.

2. An antimicrobial material as defined in Claim 1, wherein n has a value of 12 to 22 and X is chloride.

3. An antimicrobial material as defined in Claim 1 which has the unit formula

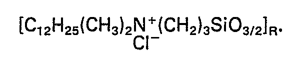

4. An antimicrobial material as defined in Claim 1 which has the unit formula

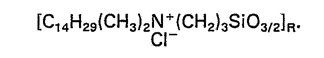

5. An antimicrobial material as defined in Claim 1 which has the unit formula

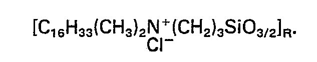

6. An antimicrobial material as defined in Claim 1 which has the unit formula

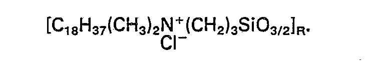

7. The antimicrobial material of any of Claims 1 to 6, wherein the silsesquioxane is combined with a cream base prior to contact with the microorganism.

8. The antimicrobial material Claim 7 of wherein the silsesquioxane and cream base also contain a medicament.

9. A polymeric silsesquioxane having the unit formula 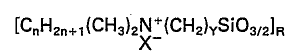 wherein n has a value of 8 to 22, y has a value of 1 to 4, R has a value such that the silsesquioxane is an insoluble friable solid and X is chloride, bromide or carboxylate radical, for elaboration into an antimicrobial contact material for inhibiting the growth of microorganisms.

## Patentansprüche

1. Unlösliches, bei Berührung das Wachstum von Bakterien hemmendes Material enthaltend ein Silsesquioxan der Einheitsformel 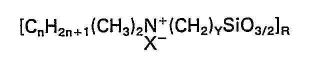 in der n einen Wert von 8 bis 22, y einen Wert von 1 bis 4 haben und R einen solchen Wert hat, daß das Silsesquioxan ein unlöslicher krümliger Feststoff ist und X Chlorid, Bromid oder eine Carboxylgruppe ist.

2. Antimikrobielles Material nach Anspruch 1, dadurch gekennzeichnet, daß n einen Wert von 12 bis 22 hat und X Chlorid ist.

3. Antimikrobielles Material nach Anspruch 1, dadurch gekennzeichnet, daß es die Einheitsformel 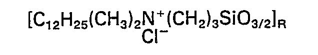 hat.

4. Antimikrobielles Material nach Anspruch 1, dadurch gekennzeichnet, daß es die Einheitsformel 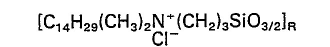 hat.

5. Antimikrobielles Material nach Anspruch 1, dadurch gekennzeichnet, daß es die Einheitsformel 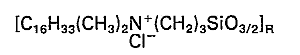 hat.

6. Antimikrobielles Material nach Anspruch 1, dadurch gekennzeichnet, daß es die Einheitsformel

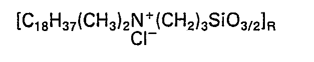

7. Antimikrobielles Material nach jedem der Ansprüche 1 bis 6, dadurch gekennzeichnet, daß das Silsesquioxan vor dem Kontakt mit Mikroorganismen mit einer Salbengrundlage kombiniert ist.

8. Antimikrobielles Material nach Anspruch 7, dadurch gekennzeichnet, daß das Silsesquioxan und die Salbengrundlage noch ein Medikament enthalten.

9. Polymeres Silsesquioxan der Einheitsformel 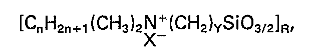 in der n einen Wert von 8 bis 22, y einen Wert von 1 bis 4 haben und R einen solchen Wert hat, daß das Silsesquioxan ein unlöslicher krümeliger Feststoff ist und X Chlorid, Bromid oder eine Carboxylgruppe ist, zum Einarbeiten in ein antimikrobielles Kontaktmaterial zum Verhindern des Wachstums von Mikroorganismen.

## Revendications

1. Une matière anti-microbienne, par contact, insoluble qui comprend un silsesquioxane ayant la formule unitaire 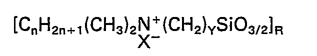 où n vaut de 8 à 22, y vaut de 1 à 4, R a une valeur telle que le silsesquioxane soit un solide friable insoluble et X est un radical chlorure, bromure ou carboxylate.

2. Une matière anti-microbienne selon la revendication 1, dans laquelle n vaut de 12 à 22 et X est le radical chlorure.

3. Une matière anti-microbienne selon la revendication 1, qui a la formune unitaire

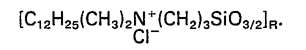

4. Une matière anti-microbienne selon la revendication 1, qui a la formule

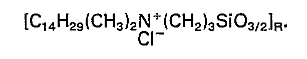

5. Une matière anti-microbienne selon la revendication 1, qui a la formule

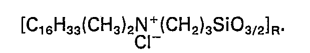

6. Une matière anti-microbienne selon la revendication 1, qui a la formule

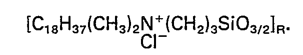

7. La matière anti-microbienne de l'une quelconque des revendications 1 à 6 dans laquelle le silsesquioxane est combiné avec une base de crème avant mise en contact avec le micro-organisme.

8. La matière anti-microbienne de la revendication 7, dans laquelle le silsesquioxane et la base de crème contiennent aussi un médicament.

9. Un silsesquioxane polymère ayant la formule unitaire 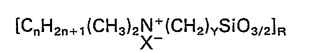 où n vaut de 8 à 22, y vaut de 1 à 4, R a une valeur telle que le silsesquioxane soit un solide friable insoluble et X est un radical chlorure, bromure ou carboxylate, pour l'élaboration d'une matière antimicrobienne par contact en vue d'inhiber la croissance de micro-organismes.
